# EUROPEAN PATENT SPECIFICATION

(11) **EP 2 311 340 B1**
(45) Date of publication and mention of the grant of the patent: **28.10.2015**
(21) Application number: 08800540.0
(22) Date of filing: 28.08.2008
(51) Int. Cl.: A43B 7/14, A43B 7/06, A43B 13/00, A61H 39/04, A61M 37/00, A61L 9/12, A43B 1/00, A61M 35/00, A43B 17/10

(54) **MEDICAL FOOTWEAR**
MEDIZINISCHE FUSSBEKLEIDUNG
CHAUSSURES MÉDICALES

(30) Priority: 23.07.2008 CN 200810134230
(43) Date of publication of application: 20.04.2011
(73) Proprietor: Wu, Liji, Inner Mongolia 010011 (CN)
(72) Inventor: Wu, Liji, Inner Mongolia 010011 (CN)
(74) Representative: Algemeen Octrooi- en Merkenbureau B.V.
(86) International application number: PCT/CN2008/001542
(87) International publication number: WO 2010/009582

(56) References cited:
- WO-A1-94/23766
- WO-A1-2007/010583
- WO-A2-2006/035469
- CN-A- 1 127 617
- CN-A- 1 142 341
- CN-U- 2 096 239
- CN-Y- 2 631 500
- CN-Y- 201 076 056
- DE-U1- 29 605 413
- GB-A- 2 350 279
- KR-A- 20080 010 057
- US-A- 3 417 494
- US-A- 5 732 485

## Description

### Technical field

This invention involves a kind of medical footwear i.e. shoes for pharmacotherapy, especially involves leather shoes, cotton padded shoes, sandals, slippers and various kinds of shoes.

### Background

Following the flying speed of society development, the people's rhythm of life is turning more and more rapid, in addition, the working intensity is increasing, and leading to less and less time available for exercise. The accumulation of the adverse factors year by year facilitates initiation of diseases. Therefore, present techniques provided people with various kinds of shoes for pharmacotherapy, in an attempt to improve the people's health status, and to prevent various chronic diseases.

However, the present shoes for pharmacotherapy are a simple combination of medicines and shoes, the sets positioning the medicines, positioning ways, as well as the composition of the medicines have not carefully examined, therefore it is difficult to achieve the anticipated effects.

US 5,732,485 relates to a deodorizer for masking undesirable odors in a shoe by the controlled release of fragrance lasting over a normal day's wear, comprising: a) a retainer having an opening defined by an edge, b) a pressure sensitive adhesive for securing said retainer to the interior surface of a shoe, c) a patch comprising a carrier, fragrance and polymer.

US 3,417,494 relates to an insole for boots or shoes comprising a two-layered article, the bottom layer being made of flexible material impervious to moisture and the top layer being made of a flexible, moisture-absorbing material, with a plurality of capsules of a volatile liquid, wherein the capsules themselves are made so that when the insole is placed in a shoe and the wearer steps on it, the capsules will rupture thus releasing the liquid which then contacts the wearer's feet. The bottom layer and top layer are cemented together throughout their entire area except for an area located in the ball portion and an area located in the heel portion, and pockets are formed in which are placed a plurality of capsules.

WO2006/035469 shows a kind of shoes (6) for pharmacotherapy, at least one trough is configured in their insoles (100), in which arbitrary numbers of containers (101, 110, etc...) of arbitrary geometries are placed, said containers are drilled with holes or are covered with fabric from which medicines are leaked out, wherein said containers are loaded with medicinal compositions having pharmacotherapy effect, which comprises at least one of the following categories: powdered preparations, liquid preparations and essential oil compositions.

### Summary of the invention

In light of the shortcomings of the present techniques, the aim of this invention is to provide a kind of medical footwear (i.e. shoes for pharmacotherapy), in order to promote unobstructed blood circulation in the wearers' feet without affecting their daily work, to treat or prevent various kinds of chronic diseases.

To realize the abovementioned aims, the embodiment of this invention is as follows:
A kind of medical footwear, wherein at least one trough (i.e. concave recess) is configured in their insoles, arbitrary numbers of containers with arbitrary geometries are placed in these troughs and bottom brackets (i.e. bottom supports) are equipped on the bottoms of said containers. The containers are drilled with holes or they are covered with fabric from which the medicines are leaked out. Said containers are loaded with medicinal compositions having pharmacotherapy effect, which comprises at least one of the following categories: powdered preparations, liquid preparations and essential oil compositions.

In a preferred embodiment of the invention, the said containers are comprised of the perforated upper lids with inner buttoning flanges or inner arcform buttoning flanges and the bottom cases with outer buttoning flanges or outer arcform buttoning flanges, and the said upper lids locked down onto the said bottom cases.

In a preferred embodiment of the invention, the said containers are comprised of the retaining rings with inner buttoning flanges or inner arcform buttoning flanges and the bottom cases with outer buttoning flanges or outer arcform buttoning flanges, and the said retaining rings locked down onto the said bottom cases. The said retaining rings fasten at least one layer of fabric onto the openings of the bottom cases.

In the present the invention, the bottoms of the containers are having at least one bottom bracket of container to avoid the containers being displaced or dislocated due to the subsidence by trample. The bottom bracket of container is arranged below the part of sole where is corresponding to the container located. The top of the bottom bracket of container reaches to the bottom surface of the sole. The thickness of the bottom bracket of container can vary depending on the thickness of the sole. The container and the bottom bracket of container can choose any material to be made into a round shape, a square shape, an unusual shape or any other shape with any volume and any size.

In a preferred embodiment of the invention, the said powdered preparations, liquid medicinal preparations include one prepared with single component of borneol, the weight ratio of borneol is in the range from 1 to 9.

In a preferred embodiment of the invention, all of the said powdered preparations, liquid preparations include one component borneol, compounded with medicines including clove, amomum cardamomum, angelica, radices zedoariae, radix angelicae and lignum cinnamomi camphorae, or are prepared with any one of the above medicine compounded solely with borneol. The weight ratios of the said medicinal materials borneol, clove, amomum cardamomum, angelica, radices zedoariae, radix angelicae and lignum cinnamomi camphorae are in the range from 1 to 9, respectively.

In a preferred embodiment of the invention, the method to prepare said powdered preparations, liquid preparations includes the following procedures:
①weigh out of the materials: the respective materials are weighed out according to the compositional weight ratio;
②the respective said materials are carefully sorted out, ground into fine powder and screened to obtain the powdered drug materials;
③borneol is solely added into the powdered drug materials to obtain said single powdered preparation; mixed powdered preparations can be prepared by mixing respective single powdered preparation if needed.
④the above powdered preparations, apart from borneol, are soaked in glacial acetic acid aqueous solution, infiltrated and extracted to obtain stock extract solutions respectively, subsequently borneol ground into fine powder is added into single stock extract solutions to obtain single liquid preparation; mixed liquid preparations can be prepared by mixing single liquid preparation with each other if needed.

In a preferred embodiment of the invention, the components of said essential oil compositions include clove oil, eugenol, eugenol type basil oil, eucalyptus oil and borneol, their weight ratios are in the range from 1 to 9, respectively.

In a preferred embodiment of the invention, the preparation method of said essential oil compositions includes the following procedures:
①weigh out of the materials: the respective said essential oil materials are weighed out according to the compositional weight ratio;
②each of the said essential oil materials is added with borneol, respectively, to obtain single essential oil composition, mixed essential oil compositions can be prepared by mixing with each other if needed.

In a preferred embodiment of the invention, after mixed up of the said medicament forms, liquid preparations and essential oil compositions, they can be applied by dropping into the said empty containers; the mixtures of said dosage forms of liquid preparations and essential oil compositions can also be applied by dropping into the containers containing said powdered preparations; each of the said medicament forms, liquid preparations and essential oil compositions, can also be applied by dropping, respectively, into the containers containing said powdered preparations.

In comparison with the present techniques, the beneficial effects of this invention are:
1. In this invention the powdered preparations, liquid preparations and essential oil compositions are placed into containers, which have perforated upper lid or are covered with cloth to enable the dissipation and subsequent functioning of the vapours of the powdered preparations, liquid preparations and essential oil compositions.
2. In this invention the powdered preparations, liquid preparations and essential oil compositions can be applied separately to exert pharmacotherapy functions, they can also be applied in compositions to exert stronger and better pharmacotherapy functions.

### Brief Description of the Drawings

Figure 1 is the stereogram of one of the medical footwear;
Figure 2 is the stereogram of an insole;
Figure 3 is the cross-section view of an insole;
Figure 4 is the stereogram of the containers placed on an insole;
Figure 5 is the cross-section view of a container placed on an insole;
Figure 6 is the stereogram of the bottom brackets for the containers on the sole;
Figure 7 is the cross-section view of the bottom brackets for the containers on the sole;
Figure 8 and 9 are the combined- and exploded view of one of the ways of implementation of the container, respectively;
Figure 10 and 11 are the combined- and exploded view of another way of implementation of the container, respectively;
Figure 12 and 13 are the combined- and exploded view of still another way of implementation of the container, respectively;
Figure14 and 15 are the combined- and exploded view of still another way of implementation of the container, respectively.

### Detailed description of the embodiments

For better understanding of the shapes, structures and features in this invention, following is the detailed illustration by means of preferable test examples accompanied with figures. The practical proportions may be changed upon drawing of the said figures for the sake of clarity of the structures in this invention, this should not be understood as restrictions to the application of this invention.

This invention is to install the mechanism of physical therapy as well as structural design of pharmacotherapy into non-padded as well as padded shoes, sandals, slippers and all kinds of shoes.

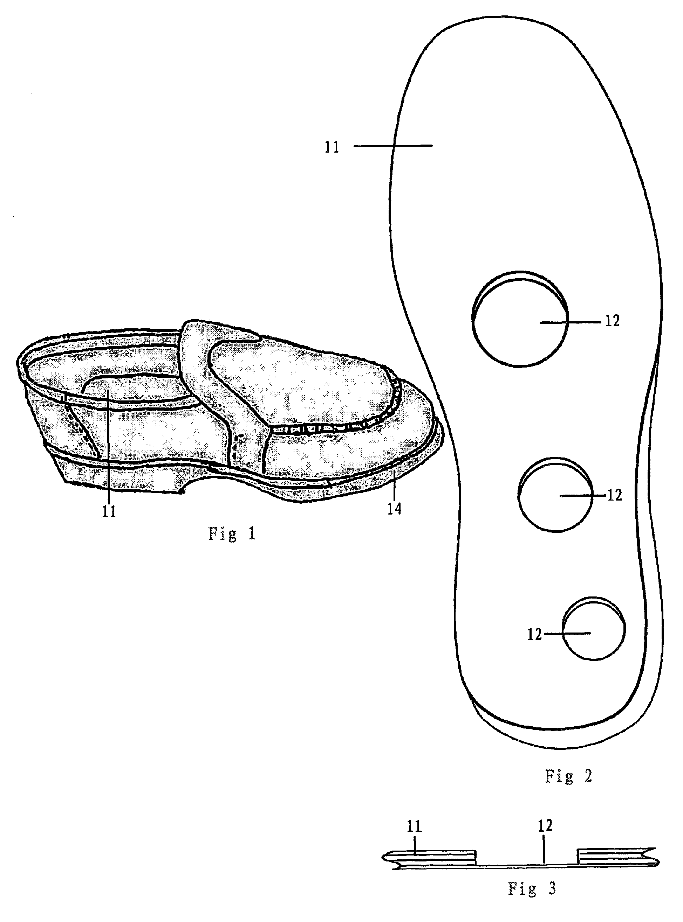

Shown in Figures 1 to 7 are the stereo-views of the medical footwear (i.e. shoes for pharmacotherapy), said medical footwear are composed of insole 11, on which at least one trough (i.e. concave recess) 12 is configured, containers 13 are placed in troughs 12, on the bottoms of the containers 13, bottom brackets of containers (i.e. bottom support) 15 are equipped on the sole 14 whose positions are corresponding to those of the troughs 12.

Shown in Figures 2 and 3 are the stereo- and cross-sectional views of the insole 11, in said insole 11, troughs 12 are configured.

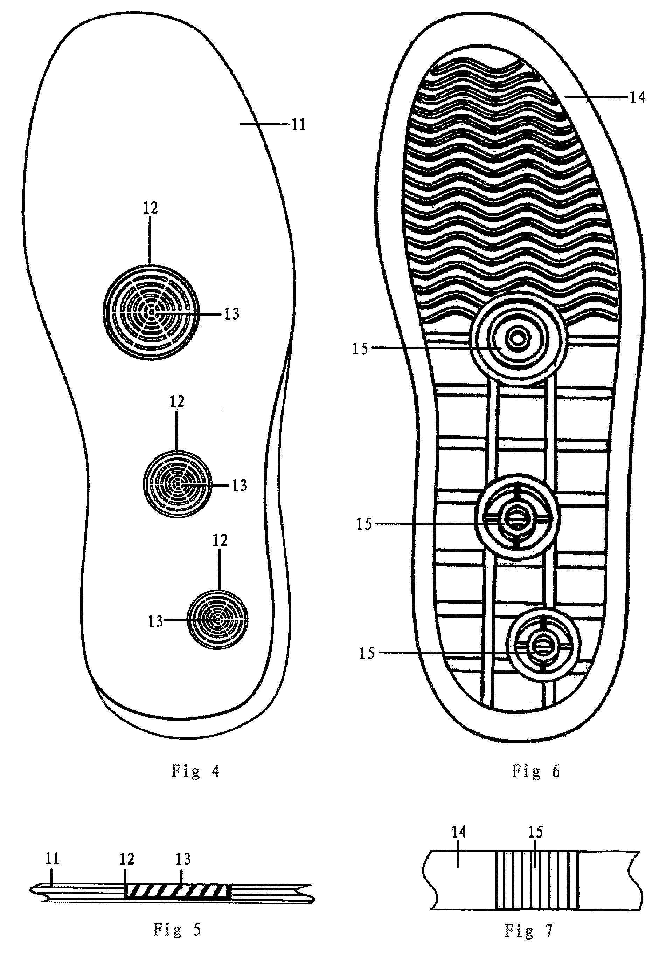

Shown in Figures 4 and 5 are the stereo- and cross-sectional views of the insole 11 placed with containers 13, which are put into troughs 12 in said insole 11.

Shown in Figures 6 and 7 are the stereo- and cross-sectional views of the sole 14 and bottom brackets of containers 15, said sole 14 is equipped with bottom brackets of containers 15, which are under the location where the containers 13 are placed in the insole 11, to prevent the containers 13 from being displaced or dislocated due to the subsidence caused by trample. It is preferable that the top of the bottom bracket of containers 15 reaches to the bottom surface of the insole (11).

The thickness of the bottom bracket of containers 15 can vary depending on the thickness of the sole 14. The containers 13 and the bottom bracket of containers 15 can choose any material to be made into a round shape, a square shape, an unusual shape or any other shape with any volume and any size.

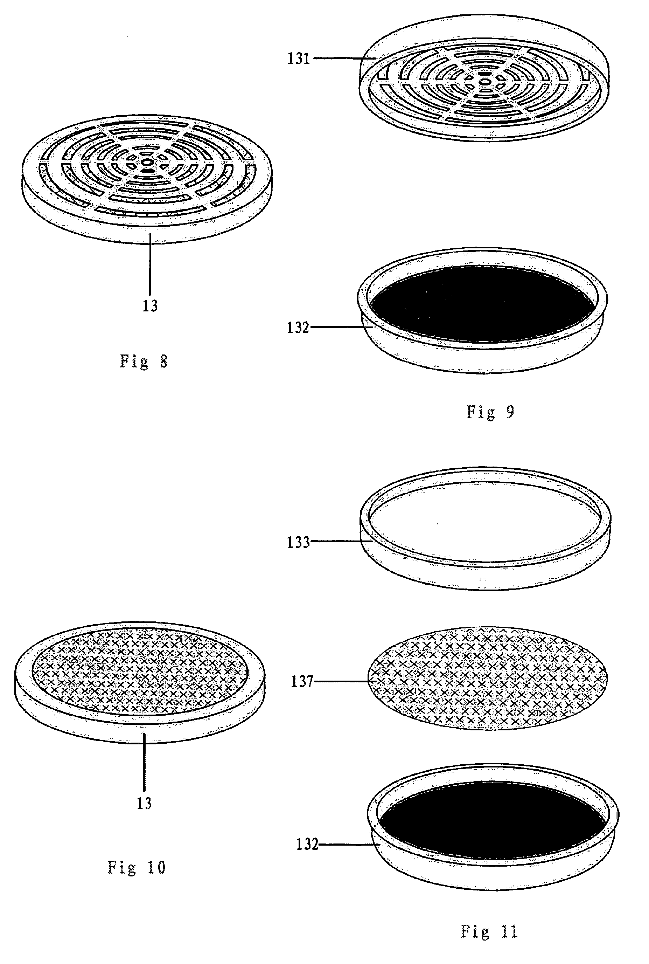

Shown in Figure 8 and 9 is said container 13, which is comprised of the perforated upper lid 131 with an inner buttoning flange locked down onto the bottom cases 132 with an outer buttoning flange.

Shown in Figure 10 and 11 is said container 13, which is comprised of a retaining ring with an inner buttoning flange and a bottom case with outer buttoning flange, the said retaining ring 133 fasten at least one layer of fabric 137 onto the opening of the bottom case 132.

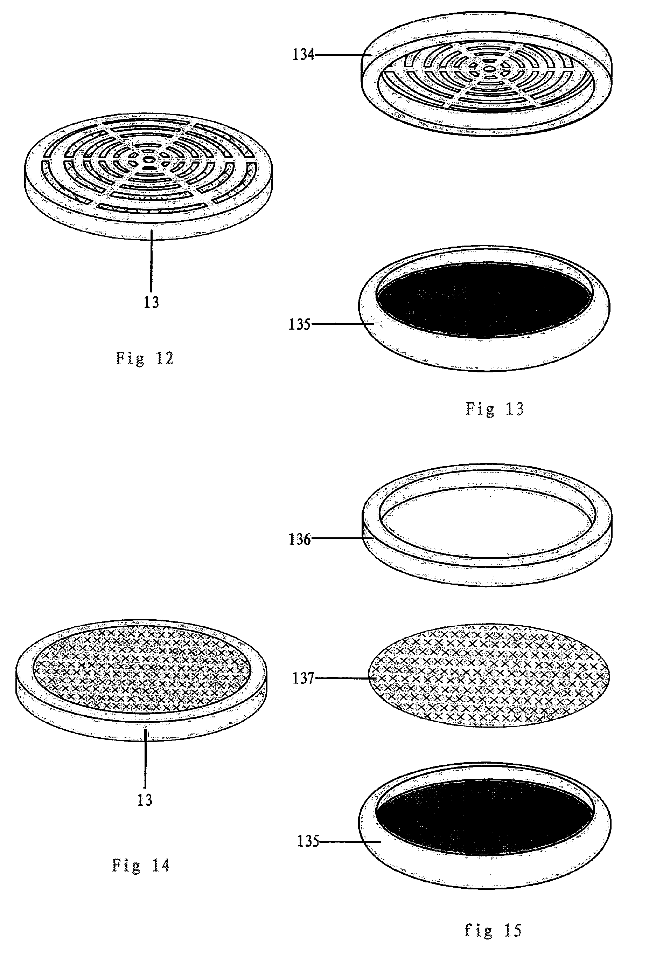

Shown in Figure 12 and 13 is said container 13, which is comprised of the perforated upper lid 134 with an arcform inner buttoning flange locked down onto the bottom cases 135 with an arcform outer buttoning flange.

Shown in Figure 14 and 15 is said container 13, which is comprised of a retaining ring 133 with an arcform inner buttoning flange and a bottom case 132 with arcform outer buttoning flange, the said retaining ring 133 fasten at least one layer of fabric 137 onto the opening of the bottom case 132.
the containers 13 is drilled with holes, or they are covered with fabric from 137 which the medicines are leaked out, said containers 13 is loaded with medicinal compositions having pharmacotherapy effect, which comprises at least one of the following categories: powdered preparations, liquid preparations and essential oil compositions.

Wherein the component of said powdered preparation, liquid preparation can include borneol alone.

In this case the method to prepare said powdered preparation is as follows: borneol is carefully sorted, ground into fine powder and sieved to obtain said powdered preparation.

And the method to prepare said liquid preparation is as follows: borneol is carefully ground into fine powder, added into aqueous solution of glacial acetic acid to obtain said liquid preparation.

The component of said powdered preparations or liquid preparations can also include other adjuvant constituents, e.g., clove and borneol, the weight of each material is in the range of 1∼9, respectively.

Following is the preparation method of said powdered preparation, including procedures stated below:
①weigh out of the materials: the respective materials are weighed out according to the compositional weight ratio, e.g., weigh out 1 weight portion of clove and 9 weight portions of borneol, or 9 weight portions of clove and 1 weight portion of borneol.
②each of the material is carefully sorted, ground into fine powder and sieved to obtain powder of each material;
③powders of all the materials are mixed up evenly to obtain said powdered proparation.

Following is the preparation method of said liquid preparation, including procedures stated below:
①weigh out of the materials: the respective materials are weighed out according to the compositional weight ratio, e.g., weigh out 1 weight portion of clove and 9 weight portions of borneol, or 9 weight portions of clove and 1 weight portion of borneol.
②each of the material is separately ground into fine powder;
③one of the materials, clove powder is soaked in glacial acetic acid aqueous solution and subsequently infiltrated and extracted to obtain said stock extract solution;
④finely ground borneol is added into stock extract solution to obtain said liquid preparation.

In the preferred practical embodiment of this invention, to prepare said powdered preparations or liquid preparations, other material components can be added, the material components added include clove, amomum cardamomum, angelica, radices zedoariae, radix angelicae and lignum cinnamomi camphorae, the weight ratios of the said materials are in the range from 1 to 9, respectively.

Following is the preparation method of said powdered preparation, including the procedures stated below:
①weigh out of the materials: the respective materials are weighed out according to the compositional weight ratio, e.g., weigh out 9 weight portions of borneol, 1 weight portion of clove, 1 weight portion of amomum cardamomum, 1 weight portion of angelica, 1 weight portion of radices zedoariae, 1 weight portion of radix angelicae and 1 weight portion of lignum cinnamomi camphorae; or 1 weight portions of borneol, 9 weight portion of clove, 9 weight portion of amomum cardamomum, 9 weight portion of angelica, 9 weight portion of radices zedoariae, 9 weight portion of radix angelicae and 9 weight portion of lignum cinnamomi camphorae.
②each of the material is carefully sorted, ground into fine powder and sieved to obtain powder of each material;
③each of the powders of all the medicinal materials is added with borneol to obtain said single powdered preparation. Single powdered preparations are mixed up if mixed powdered preparations are needed.

Following is the preparation method of said liquid preparations, including the procedures stated below:
①weigh out of the materials: the respective materials are weighed out according to the compositional weight ratio, e.g., weigh out 9 weight portions of borneol, 1 weight portion of clove, 1 weight portion of amomum cardamomum, 1 weight portion of angelica, 1 weight portion of radices zedoariae, 1 weight portion of radix angelicae and 1 weight portion of lignum cinnamomi camphorae; or 1 weight portions of borneol, 9 weight portion of clove, 9 weight portion of amomum cardamomum, 9 weight portion of angelica, 9 weight portion of radices zedoariae, 9 weight portion of radix angelicae and 9 weight portion of lignum cinnamomi camphorae.
②each of the material is carefully sorted, ground into fine powder and sieved to obtain powder of each material;
③each of the powders of all the medicinal materials is added with borneol to obtain said single powdered preparation. Single powdered preparations are mixed up if mixed powdered preparations are needed.
④said medicinal powdered preparations, apart from borneol, are separately soaked in glacial acetic acid aqueous solution and subsequently infiltrated and extracted to obtain said stock extract solution of the materials, respectively, each of the stock extract solutions is added with finely ground borneol to obtain said single liquid preparation, mixed liquid preparations can be prepared by mixing up single liquid preparation if needed.

Said essential oil compositions include clove oil, eugenol, eugenol type basil oil, eucalyptus oil and borneol, their weight ratios are in the range from 1 to 9, respectively.
①weigh out of the materials: the respective materials are weighed out according to the compositional weight ratio, e.g., weigh out 9 weight portions of borneol, 1 weight portion of clove oil, 1 weight portion of eugenol type basil oil, 1 weight portion of eugenol, 1 weight portion of eucalyptus oil; or weigh out 1 weight portion of borneol, 9 weight portions of clove oil, 9 weight portions of eugenol type basil oil, 9 weight portions of eugenol, 9 weight portions of eucalyptus oil.
②borneol is separately ground into fine powder;
③each of the said essential oil materials is added with borneol, respectively, to obtain said single essential oil composition. Single essential oil compositions are mixed up if mixed essential oil compositions are needed.

When using the said medical footwear, two medicament forms, said liquid preparations and essential oil compositions, can be dropped into empty containers after being mixed up; two medicament forms, said liquid preparations and essential oil compositions, can also be applied by dropping into said containers containing powdered preparations separately; two medicament forms, said liquid preparations and essential oil compositions, can also be applied by mixing up them and subsequently dropping the mixture into said container containing powdered preparations.

As stated above, the insole 11 of said medical footwear is configured with containers 13 containing powdered preparations, liquid preparations, essential oil compositions. The medicaments in the container 13 offer the wearers with pharmacotherapy, the medicinal function can be percutaneously absorbed from the feet to promote unobstructed circulation and microcirculation and to activate cells, and finally, to contribute to the pharmacotherapy and healthcare of the human body. The said shoes are suitable for the patients with ischemic chronic diseases such as diabetes and its complications, hypertension, coronary disease, bradycardia, cerebral infarction, cerebral thrombosis, postencephalitis, cervical spondylosis, pathological changes of vertebrae lumbales, scapulohumeral periarthritis, rheumatosis, rheumatoid disease, gout, debility of loin and legs, insomnia, constipation, hyperplasia of prostate, bronchitis, allelgic asthma, allergic rhinitis, neurodermatitis, eczema, drug rash, anaemia, gastritis, enteritis, nephropathy, gynopathy, they are also suitable for the treatment of several diseases simultaneously with high rate of rehabilitation, they are safe and practical, easily applicable.

The aforementioned only offers an optimal implementation of this invention, many small adjustments can be made in practice, for instance:
The troughs 12 and the containers 13 in the insole 11, and the bottom brackets of containers 15 on the sole 14 can choose any material to be made into a round shape, a square shape, an unusual shape or any other shape with any volume and any size, which are not shown as the changes of structure are simple and can be easily understood.

The above example does not sum up all the examples of this invention. On the basis of this invention, by carrying out various changes and combinations, for example, by doing technical redressal and recombination of the designing and structures of this invention to non-padded as well as padded shoes, sandals, slippers and various kinds of shoes, the technicians working in this field can produce different types of medical footwear, without creative work, nevertheless, these are all within the protective range of this invention.

The above explanation is only illustrative and not restrictive, the technicians working in this field understand that under the condition of not disengaging from the spirit and scope defined by the claims, many modifications, changes and equivalents can be made, nevertheless, these are all within the protective range of this invention.

Finally, the beneficial effects of this invention are described in accordance with modern medicinal theory:
When biological currents are abundant, bone density will increase and the hematopoietic function of the marrow stem cells be strengthened:
   The skeleton is the framework of human body, marrow stem cells are the hematopoietic factory of the red cells, white cells and platelets.

Putting on the said shoes, they will offer the feet of the wearers with pharmacotherapy measures, the pharmacological effects of the medications in the shoes will be percutaneously absorbed from the feet to activate cells and to promote unobstructed circulation and microcirculation, and finally, to contribute to the pharmacotherapy of the human body. Then the red cells will be more agile and active, they will be supplied with abundant reinforcement of biologic current, the velocity at which they carry oxygen, curl and move helically forward will increase. The red cells will dredge swiftly the capillary vessel bed inside bones and endosteum, resulting in unobstructed, vigorous and smooth blood circulation in bones and endosteum.

In this way the problems of microcirculation obstruction inside the bones and endosteum, as well as ischemia and anoxia can be essentially eliminated.

When the blood circulation inside the bone is unobstructed, vigorous and smooth, the bone will obtain ample supply of ossein fiber bundles as well as vitamin A, D and C, to promote the formation of bone matrix.

In this way, precipitation of the osteoid and calcium salts onto the bone matrix can be facilitated, bone density be increased, and the waists and the legs of the wearers be strengthened. When they go upstairs of walk a long way, they will not feel tired and lack of strength, as well as sore waist and aching legs. The problem of clonus frequently occur in the middle aged and the old due to acalcerosis and anoxia is thereby being eliminated.

As the red cells dredge capillary vessel bed inside bones and endosteum, they transfer the biologic current they carry with them to the marrow stem cells, i.e. the red marrow.

Thus the marrow stem cells will be supplied with ample amount of biologic current, as well as blood and oxygen. The hematopoietic function of the marrow stem cells will be strengthened, producing mature red cells, white cells as well as platelets in due course, and keep the numbers of these blood cells produced at normal amounts.

Therefore, putting on said shoes especially keeps the hematopoietic physiological function of the bones and marrow stem cells of the middle aged and the old at an active and thriving status for extensive period of time. The middle-aged and the old will get rid of various inflammations and galls, the problem of feebleness in their waists and legs after putting on said shoes, they are forceful and agile in their waists and legs, their physique and immunity strengthened, by and large they will not catch cold. Besides, putting on said shoes will postpone ageing and prolong life of the wearers.

To validate the therapeutic and healthcare functions of the medical footwear, the applicant offer a document showing the results of the clinical trials of this invention in the hospitals in Innermongolia autonomous region:

### I. Clinical trials in the Innermongolia autonomous region hospital

### Type of disease: hypertension

Starting and finishing dates: 5. Dec. 1995-15. Jan. 1996.

No. of cases: Male: 20, female: 10, sum of patients: 30, age: 35-65.

Source of the patients: All patients were diagnosed by out-patient department to be hypertensive. During the observation period, apart from patients of phase I hypertension who stopped taking antihypertensive drugs, all of the patients of phase II hypertension continued to take drugs, the patients measured blood pressure regularly, twice a day, in the course of observation, patients whose blood pressure decreased took reduced amount of antihypertensive drugs or ceased to take the drugs. Among 30 patients, 3 were phase II patients.

Clinical effectiveness rating standard: (effectiveness is rated into 4 classes: healed, significantly effective, effective, ineffective, other execution refers to this)
1, significantly effective: ①diastolic pressure lowered by 1.33kPa (10mmHg) or more; ②diastolic pressure had not been lowered to normal, yet lowered by 2.67kPa or more.
2, effective: ①diastolic pressure lowered by less than 1.33kPa, yet lowered to normal. ②diastolic pressure lowered by 1.33-2.53kPa than before therapy, yet had not been lowered to normal. ③diastolic pressure lowered by 4.00kPa than before therapy.
3, ineffective: those failed to reach the above standard.

Typical case: Patient name: Han Zhongchang, Male, Age: 58, BW: 76kg, officer serving in foreign trade, diagnosed to be hypertensive for 6 years, had taken antihypertensive discontinuously since. Symptom: dizziness, feeling headache, highest blood pressure reached 180/120mmHg. On 5, Dec. 1995 the patient began to put on said shoes, the blood pressure lowered to normal the second day (the patient measured blood pressure 3-4 times as a person in his family was medical doctors), after ceasing to put on said shoes for 3 days, his blood pressure increased slightly, he then put on said shoes and stopped to take antihypertensive. According to the self report of the patient, symptoms of dizziness and feeling headache improved evidently. Cardiogram examination: shift down of the S-T segment improved then before. Blood and urine routine were normal.

Clinical effectiveness statistics:
Phase I hypertension: 9 cases

| | |
|---|---|
| significantly effective | 0 cases, accounted for 0% of the total |
| effective | 7 cases, accounted for 77.8% of the total |
| ineffective | 2 cases, accounted for 22.2% of the total |

Phase II hypertension: 18 cases

| | |
|---|---|
| significantly effective | 2 cases, accounted for 11.1% of the total |
| effective | 14 cases, accounted for 77.8% of the total |
| ineffective | 2 cases, accounted for 11.1% of the total |

Phase III hypertension: 3 cases

| | |
|---|---|
| significantly effective | 1 case, accounted for 33.3% of the total |
| effective | 2 cases, accounted for 66.7% of the total |
| ineffective | 0 cases, accounted for 0% of the total |

Total No. of cases. : 30 total effectiveness is 86.7%

| | |
|---|---|
| significantly effective | 3 cases, accounted for 10% of the total |
| effective | 23 cases, accounted for 76.7% of the total |
| ineffective | 4 cases, accounted for 13.3% of the total |

Conclusion to the clinical trials: From the clinic observation to 30 hypertensive patients, said shoes have a certain curative effect to improve the hypertensive symptom and decrease the blood pressure, the blood pressure of some mild hypertensive patients could be lowered to normal without taking antihypertensive, several moderately hypertensive patients stopped taking antihypertensive due to lowering of blood pressure, and the dizziness and headache normally seen in the hypertension improved evidently.

### II. Clinical trials in the Innermongolia autonomous region hospital

### Disease: diabetes

Starting and finishing dates: 5.Dec.1995-15. Jan.1996, 2 Courses of treatment, 40 days.

No. of cases: diabetes 30 [Insulin-dependent diabetes mellitus (IDDM) 5, Non-insulin-dependent diabetes mellitus (NIDDM) 25]. Male: 15, female: 15, Max. age: 69, Min. age: 25.

Source of the patients: All the 30 patients were diagnosed as diabetics in accordance with the diagnostic standard set down by WHO Expert Committee on Diabetes Mellitus. The clinic patient from Hohehot Municipality cooperated with the doctors by accepting follow-ups. Among 30 cases, 25 were mild, 5 were moderate. Among 25 mild patients, 5 depended on diet, 20 took oral hypoglycemic agents. 5 moderate patients received insulin therapy, yet all the 30 patients were able to participate in normal work, suffering from no severe acute or chronic complications.

Clinical effectiveness rating standard: (effectiveness is rated into 4 classes: healed, significantly effective, effective, ineffective, other execution refers to this)
1, significantly effective: laboratory assay showed significant decrease of the blood sugar and urine sugar, other symptoms and physical signs improved evidently.
2, effective: laboratory assay showed some degree of decrease of the blood sugar and urine sugar, other symptoms and physical signs improved evidently.
3, ineffective: laboratory assay showed no decrease of the blood sugar and urine sugar, other symptoms and physical signs had no evident improvement.

Typical case: patient name Li Suohuan, Male, 58, BW: 75kg. Symptoms: polydipsia, polyuria, thirst, became thin for more than 10 months. Diagnosed diabetic in the out-patient department of this hospital, treated with control of eating and drinking and taking small amount of oral hypoglycemic agents. Clinic symptom and control of blood sugar and urine sugar were not satisfactory. Before trying to put on said shoes, the patient stopped taking oral hypoglycemic agents, blood sugar assay 10.8mmol/L, complaining thirst, feebleness and polydipsia. After first course of treatment using said shoes, blood sugar assay 7.1 mmol/L, urine sugar (-) and above, self feeling changed for the better; after second course of treatment, blood sugar lowered to normal range (6.15 mmol/L), the whole body's self feeling is all right. The patient told us very excitedly that this kind of shoes were worth popularization. It was comfortable to put on them, he would like to make propaganda for us.

### Clinical effectiveness statistics:

diabetes: 30 cases

| | |
|---|---|
| Significantly effective: | 12 cases, accounted for 40% of the total |
| Effective: | 15 cases, accounted for 50% of the total |
| Ineffective: | 3 cases, accounted for 10% of the total |

Total effectiveness: 90%

Conclusion to the clinical trials:
1, from the clinic observation to 30 patients, it can be concluded that it is certain the said shoes have some degree of therapeutic effect on diabetes. It is especially suitable on the treatment of mild diabetes, however, it should be reminded that it is wrong to think one-sidedly that no other treatment is needed after putting on said shoes, as the modern treatment of diabetes is comprehensive.
2, not only the said shoes have therapeutic effect, but also it is very good health care product for diabetic patient, putting on them is comfortable, feeling soft and not sense of pain, they have protective effect on diabetic patients.
3, no adverse effect was found in the course of application of the said shoes.

### III. Clinical trials in the Innermongolia autonomous region hospital

### Disease: coronary heart disease

No. of cases. : 30 in total, among which 20 males, 10 females, age: in the range of 36-65. Mild patients: 10, moderate: 19, severe: 1.

Source of the patients: all were out-patients diagnosed in the hospital to suffer from coronary heart disease.

Clinical effectiveness rating standard: (effectiveness is rated into 4 classes: healed, significantly effective, effective, ineffective, other execution refers to this)
1. Significantly effective: 80% improvement of clinical symptoms, or 0.03mv recovery of the S-T segment in cardiogram, and shallower inverted T wave than before treatment.
2. Effective: 60% improvement of clinical symptoms.
3. Ineffective: less than 60% of clinical symptom, no improvement is cardiogram.

Typical case: Name of the patient: An Zhiguo, male, 51, BW: 70.5kg, feeling headache and dizziness, feeling pain in the precordium and feebleness for more than 3 years, diagnosed coronary heart disease by using heart type-B ultrasonic and cardiogram, had been treated by taking coronary artery dilation and anticoagulating drugs, putting on the said shoes since 5. Dec. taking coronary artery dilation and anticoagulating drugs simultaneously, 3 days later headache is alleviated, 1 week later, heart-throb and pain in the precordium were alleviated evidently, 20 days later cardiogram was approximately normal, 40 days after reduced doses of coronary artery dilation and anticoagulating drugs, cardiogram was approximately normal, other symptoms essentially faded away. Result of examination: 20 days after putting on said shoes ST-T in the cardiogram improved, blood and urine routine were normal. 40 days after putting on said shoes ST-T in the cardiogram was approximately normal, blood and urine routine were normal.

### Clinical effectiveness statistics:

coronary heart disease: 30 cases

| | |
|---|---|
| Significantly effective | 10 cases, accounted for 33.3% of the total |
| Effective | 17 cases, accounted for 56.7% of the total |
| Ineffective | 3 cases, accounted for 10% of the total |

Total effectiveness: 90%

Conclusion to the clinical trials: From the clinical observation of 30 coronary heart disease patients, the said shoes had a certain degree of curative effect on the symptoms of coronary heart disease and ischemia myocardial, most of the patients felt evident alleviation of dyspnea, symptoms such as whole body feebleness alleviated evidently, some could reduce doses of coronary artery dilation and anticoagulating drugs, Some patients used to feel cold in the lower limbs owing to poor peripheral circulation, after putting on said shoes, they felt warm and comfortable in the feet although in winter. A few patients showed bleeding, on account of the anticoagulating effect of said shoes, it is advisable that the hemorrhagic patients should not use said shoes.

### IV. Clinical trials in the Innermongolia autonomous region hospital

### Disease: arteriosclerosis

Starting and finishing dates: 1. ∼ 31. Dec. 1996.

No. of cases: Total: 30, male: 25, female: 5. Age: 40-70.

Source of the cases: all are out-patients diagnosed to be arteriosclerotic patients.

Clinical effectiveness rating standard: (effectiveness is rated into 4 classes: healed, significantly effective, effective, ineffective, other execution refers to this)
1. Significantly effective: cardiagram returned to normal, fading away of various symptoms caused by arteriosclerosis.
2. Effective: improvement of cardiogram to some extent, evident alleviation of most of the symptoms caused by arteriosclerosis.
3. Ineffective: no improvement.

Typical case: name of the patient: An Zhiguo, male, 52, BW: 65.5kg, feeling dyspnea and dizziness, pain in the precordium for more than 3 years, blood assay showed increase of blood fat and blood viscosity, T wave in the cardiogram had changed, color ultrasonogram showed aortosclerosis, treated using blood-lipid lowering and anticoagulating drugs since diagnosis. Put on said shoes since early December and took the abovementioned drugs with the reduced doses simultaneously, 20 days later, dyspnea and pain in the precordium were alleviated, 40 days later, cardiogram and heart color ultrasonogram showed evident improvement, aortosclerosis essentially faded away.

### Clinical effectiveness statistics:

Arteriosclerosis: 30 cases

| | |
|---|---|
| Significantly effective: | 4 cases, accounted for 13% of the total |
| Effective: | 23 cases, accounted for 77% of the total |
| Ineffective: | 3 cases, accounted for 10% of the total |

Total effectiveness: 90%

Conclusion to the clinical trials: from clinical observation of the 30 arteriosclerotic patients, said shoes alleviated to some extent the arteriosclerotic symptom and cardiogram, most patients feeling evident alleviation of symptoms such as dyspnea, cardiopalmus, pain in the precordium, and whole body feebleness, cardiogram improved evidently. From accompanying examination, the patient had some improvements in arteriosclerotic conditions, the blood fat and blood sugar of most of the patients decreased to some extent. Some patients used to feel cold in the lower limbs owing to poor peripheral circulation, after putting on said shoes, they felt warm and comfortable in the feet although in winter.

### V.Clinical trials in the Innermongolia Sino-mongolian hospital

### Disease: chronic rheumatoid arthritis

Starting and finishing dates: 8. Dec. - 18. Jan. 1996, 41 days in total

No. of cases: Sum: 30, male: 12, female: 18. Age: 19-70, mild state of the illness: 3, moderate: 25, severe: 2.

Source of the patients: out-patients and in-patients in the hospital.

Case analysis of this group of patients showed following characteristic:
1, more than 80% of patients suffered from chronic rheumatoid arthritis, in every winter and during change of weather feeling of pain turned severer, whereas the results of assay showed no significant changes, they are by and large in normal range.
2, historically, the patients had been treated repeatedly. They had taken supplemental doses of traditional Chinese-, Western- and part of Mongolian medicines.

Clinical effectiveness rating standard: (effectiveness is rated into 4 classes: healed, significantly effective, effective, ineffective, other execution refers to this)
1. Significantly effective: chronic patients mainly judged by improvement of clinical symptoms: improvement of 75% -80% of total symptoms. For acute patients, besides improvements of clinical symptoms, (for a portion of them) the erythrocyte sedimentation and O-resistance turned normal.
2. Effective: for chronic rheumatoid arthritic patients, improvement of more than 60% of the total symptoms. For acute patients erythrocyte sedimentation and O-resistance showed alleviation.
3. Ineffective: for chronic rheumatoid arthritic patients, improvement of less than 50% of the total symptoms.

Typical case: patient name: Ao Zhanyuan, male, 56, BW: 65kg. The patient caught chronic rheumatoid arthritis 10 years ago due to catch a chill, for 10 years the patient had taken many Western-, traditional Chinese,- and Mongolian medicines for treatment of rheumatoid arthritis, yet the disease recurred. This December the patient went of the out-patient department for treatment, tried to put on the said shoes under the direction of the doctors, after a dozen days the patient felt fine, the articulations and insteps began to feel warm, he no longer suffered from pain due to the change of weather, said shoes played a role in therapeutical effect.

The patient was chronic arthritic, the assays of blood- and urine routines, erythrocyte sedimentation showed no significant abnormities, only suffered from recurrent pain when the weather changed, the result of treatment is stated above.

### Clinical effectiveness statistics:

No. of cases. : 30

| | |
|---|---|
| Significantly effective: | 6 cases, accounted for 20% of the total |
| Effective: | 21 accounted for 70% of the total |
| Ineffective: | 3 cases, accounted for 10% of the total |

Total effectiveness 90%

Conclusion to the clinical trials: From the clinical observation of 30 patients, it can be concluded that the said shoes played a role in good therapeutic effects on chronic rheumatoid arthritis. From observation to the results of two courses of treatment for more than 40 days, the total effectiveness was 90%.

The characteristics is that they improved the feelings of cold in lower limbs and pain in the articulations significantly and rapidly, they caused warm and comfortable feeling, suitable for two functions of health care and therapy simultaneously.

### VI.Clinical trials in the Innermongolia Sino-mongolian hospital

### Disease: kidney vacuity

Starting and finishing dates: 8. Dec. 1995 -18. Jan. 1996.

No. of cases. : 30 in total, male: 15, female: 15. Age: 22 in the range of 30-40, 3 in the range of 41-50, 4 in the range of 51-60, 1 older than 60.

Source of the patients: all selected from the out-patients who showed good compliance.

Clinical effectiveness rating standard: (Rated into 4 ranks: healed, significantly effective, effective, ineffective, other execution refers to this)
1, significantly effective: more than 80% of the total clinical symptoms and physical signs turned normal.
2, effective: more than 60-70% of the total clinical symptoms and physical signs turned normal.
3, ineffective: less than 50% of the total clinical symptoms and physical signs turned normal.

Typical case: patient name: Zhou Yujuan, female, 31, serving in the Law office, Huimin District, Hohehot Municipality. Medical history: After delivery 3 years ago, the patient suffered from stiffness in the waist, feebleness, felt lassitude and sour in loin and legs, hidrorrhea due to deficiencies even after light laboring, the whole body felt weak, leucorrhoea increased after menstruate. Symptom intermittently turned mild and severe, symptoms and body signs aggravated especially when tired and in bad mood. After using the said shoes, symptoms such as stiffness in the waist, feebleness, lassitude and sour in loin and legs, hidrorrhea due to deficiencies, increased leucorrhoea were alleviated evidently, after using for a month, the state of illness was basically stabilized, spirit and physical strength was mended.

### Clinical effectiveness statistics:

Kidney vacuity: 30 cases

| | |
|---|---|
| Significantly effective: | 5 cases, accounted for 16% of the total |
| Effective: | 23 cases, accounted for 76.6% of the total |
| Ineffective: | 2 cases, accounted for 6% of the total |

Total effectiveness: 93.2%

Conclusion to the clinical trials: various symptoms induced by kidney vacuity is a common ailment among those in the middle age and prime of the life, it is frequently encountered in clinic. The said shoes is developed according to traditional Chinese medicinal theory in combination with modern technologies, their therapeutic principle is guiding TCM into human main and collateral channels through the acupoints to alleviate various symptoms induced by kidney vacuity etc. meticulous observation verified that total effectiveness of said shoes to various lumbago, cold and deficiency, lumbar hyperosteogeny etc. induced by kidney vacuity was 93.2%. They have significant effect on symptoms such as stiff waist, feebleness, lassitude and sour in loin and legs and hidrorrhea etc. Their therapeutic effect is reliable, they have the advantages of functioning rapidly, having no adverse and toxic effects, easy to use, and simple to operate, it is worthwhile to promote their application to the society.

### VII. Clinical trials in the Innermongolia Sino-mongolian hospital

### Disease: neurosis

Starting and finishing dates: 8. Dec. 1995 ∼ 18. Mar. 1996.

No. of cases: 30 in total, male: 18, female: 12. Age: 22-74. Mild state of the illness: 6, moderate: 18, severe: 6.

Source of the patients: Mainly out-patients in the hospital.

Case analysis of this group of patients showed following characteristic:
1, neurotic symptoms occurred in a part of the youths, especially university and technical secondary school students and brainworker owing to age long psychentonia.
2, neurotic and neurasthenic symptoms in a part of the patients because they had the problems of senility and feebleness, difficulties in their lives and their children's difficulties in obtaining employment, etc..

Clinical effectiveness rating standard: (Rated into 4 classes: healed, significantly effective, effective, ineffective, other execution refers to this)
1. Significantly effective: improvement of 80% of the clinic symptoms.
2. Effective: improvement of 60% of the clinic symptoms.
3. Ineffective: more than 50% of the clinic symptoms were not changed.

Typical case: name of the patient: He Yongsheng, male, 54, BW: 82kg, hospitalized in 9. Dec. 1995, diagnosis: 1-neurosis, 2-neurasthenia. Main symptoms were: nervous, cardiopalmus, insomnia, dreaminess, tiredness for more than 2 years, aggravated for 2 months. Medical examinations: blood pressure: 130/90mmHg, body temperature 36oC, pulse 76/min, breath: 19/min, bodily form: endomorphy type. The patient made a trial to put on the said shoes under the guidance of a doctor of this hospital when he went to see the doctor, on the fifth day he returned to the hospital telling that the symptoms of nervousness, cardiopalmus and tinnitus etc. began to be changed for the better, he began to sleep sounder at night, after continuing to use said shoes the therapeutic effect is significant. Clinic assay were not done for patients in this group.

### Clinical effectiveness statistics:

Neurosis: 30 cases

| | |
|---|---|
| Significantly effective | 13 cases, accounted for 43.3% of the total |
| Effective | 15 cases, accounted for 50% of the total |
| Ineffective | 2 cases, accounted for 6.7% of the total |

Total effectiveness: 93.3%

Conclusion to the clinical trials: From clinic observation of 30 patients for more than 40 days, it can be concluded that the said shoes had a certain curative effect on various clinical symptoms of neurasthenia. Total effectiveness reached 93.3%. The patients generally reflect that putting on the said shoes, feet felt warm and comfortable, revivified the wearers, they were not easily felt sleepiness, as well as tiredness after walking a long way. Besides, no adverse effects were found.

### VIII.Clinical trials in the Innermongolia Sino-mongolian hospital

### Disease: chronic bronchitis

Starting and finishing dates: 1. Dec. 1996-28. Mar. 1997.

No. of cases: 60 in total, divided into observation group (30) and control group (30). Observation group, male: 20, female: 10 age: 28-67 (48.5±9.7); control group, male: 16, female: 14, age: 25-73 (average 50.2±13.3).

Source of the cases: All the cases were from the Self-diagnosing patients and in-patients from the departments of respiratory diseases, emergency and senile disease.

Clinical effectiveness rating standard: (Rated into 4 ranks: healed, significantly effective, effective, ineffective, other execution refers to this)
1. Healed: after therapy all the symptom and body signs faded away, all the physical and chemical indices return to normal completely.
2. Significantly effective: after therapy all the symptom and body signs improve more than 70%, physical and chemical indices resume more than 2/3.
3. Effective: after therapy all the symptom and body signs improve more than 30%, physical and chemical indices resume more than 1/3.
4. Ineffective: after therapy all the symptom and body signs show no improvement or improve less than 30%, physical and chemical indices resume less than 1/3.

Typical case: patient: Yang Lin, male, 58, serving in the Standing Committee of the People's Congress of suburb of Hohehot Municipality, Tel: 6957140. The main symptoms of the patient were cough and stethocatharsis, especially when get up in the morning, history of the disease: 5 years, X-ray diagnosis in this hospital was chronic bronchitis. Since Jan. 97 the patient began to use the said shoes for therapy, course of treatment was 40 days. While treated with putting on said shoes for treatment, the patient took cephalosporin capsules 3^{T} tid po, for 1 week in total. After using the said shoes and taking medicine for a week, coughing and amount of stethocatharsis reduced evidently, from 30ml each morning after getting up to 1-2 stethocatharsis, after treating for 40 days, the symptoms of coughing and stethocatharsis faded away, X ray irradiation countercheck showed evident absorption of the phlegm than before treatment, also assay of immunoglobulin improved evidently, this meant that the said shoes, coordinated with medicinal treatment, would enhance immunity of the body, improve its health status, they had a certain adjuvant curative effect on chronic bronchitis.

### Clinical effectiveness statistics:

Chronic bronchitis: observation group 30 cases

| | |
|---|---|
| Significantly effective: | 5 cases, accounted for 16.7% of the total |
| Effective: | 21 cases, accounted for 70% of the total |
| Ineffective: | 4 cases, accounted for 13.3% of the total |

Total effectiveness: 86.7%

Chronic bronchitis: control group 30 cases

| | |
|---|---|
| Significantly effective | 5 cases, accounted for 16.7% of the total |
| Effective | 20 cases, accounted for 66.7% of the total |
| Ineffective | 5 cases, accounted for 16.7% of the total |

Total effectiveness: 83.3%

### Conclusion to the clinical trials:

1, Conclusion to the clinical study on the disease mechanism:
   ① for objectively evaluating the therapeutic effect of said shoes on chronic bronchitis, a control group was designed in the experiment. The control- and observation groups were treated with the identical medicinal therapy, only in the observation group treatment with the said shoes was increased, other conditions being the same. The two groups were divided using randomization grouping method, in terms of age, sex and status of illness, between the two groups there were good comparability. Therefore the result of the clinical research was objective and reliable.
   ② clinical data showed that both the observation- and control groups have a certain therapeutic effect after treatment, comprehensive therapeutic effect analysis showed that there were no statistic significance between the rate of significantly effectiveness and total effectiveness in the two groups (all the P values>0.05), this meant that the therapeutic effect was caused by the effect of medication. However, in regard to the alleviation of stethocatharsis, gasp, nervousness, short breath and wheeze, observation groups is more favorable than control group, yet statistical significance had not reached (all the P values>0.05), this suggested that the said shoes only had a certain adjuvant therapeutic effect, most of the patients (more than 70% of the total) felt that the feet warm and comfortable, coldness in the lower limbs was improved, this suggested that said shoes helped to improve the peripheral circulation. Clinical studies showed that said shoes helped to improve some symptoms and body signs to a certain extent to types of chronic bronchitis below: cold and deficient in liver, spreen and kidney type and failure of the kidney to tonify type.
2, Conclusion to the clinical trials:
   ① comprehensive therapeutic evaluations and all of the observed indices showed no statistical significance between observation- and control groups.
   ② the said shoes have adjuvant therapeutic effects to types of chronic bronchitis below: cold and deficient in liver, spreen and kidney type, and failure of the kidney to tonify type.
   ③ said shoes have relatively evident effects of keeping feet warm and improving the peripheral circulation, they may help to promote running of QI of meridian and adjusting the equilibrium of Yin and Yang.
   ④ no adverse effects of said shoes were found in clinical studies.

### IX. Clinical trials in the Innermongolia Sino-mongolian hospital

### Disease: sequela of ischemic cerebral disease

Starting and finishing dates: Dec. 1996-May 1997.

No. of cases: stochastically divided the selected 60 patients into "pharmacotherapeutic shoes group" (30) and "control group"(30).

Pharmacotherapeutic shoes group: male: 24, female: 6, average age: 55.9±9.0. (40-75)

### Control group: male: 25, female: 5, average age: 54.6±9.3. (41-73)

Source of the cases: all were from the out-patient clinic and wards belonged to cerebral disease department, emergency department and medical department in this hospital.

Clinical effectiveness rating standard: (Rated into 4 ranks: healed, significantly effective, effective, ineffective, other execution refers to this)
1. Healed: all the symptoms and body signs faded away or returned to normal, muscle strength reached 5 scale or higher after therapy.
2. Significantly effective: symptoms and body signs improved by 70% or more, muscle strength increased by 2 scale or higher after therapy.
3. Effective: symptoms and body signs improved by 30% or more, muscle strength increased by 1 scale or higher after therapy.
4. Ineffective: symptoms and body signs not improved or improved by less than 30%, muscle strength did not increase after therapy.

Typical case: Patient: Xu Zizhen, male, 75, living in the old inner city of Hohehot Municipality. In October 1996 the patient suddernly suffered from facial paralysis and stiff tongue, inconvienence to move the right side limbs at will, head CT examination verified cerebral lacunar infarction (compounded with hypertension phase III), 4 months after drug therapy using combination of traditional Chinese and Western medicinal method, the patient still suffered from stiff tongue, headache, dizziness, chilly, feebleness of the limbs, after therapy by putting on said shoes and took Naoxinkang 3 tablets t.i.d. and Naofukang tablets 0.8g t.i.d., after treating for 20 days, symptoms of stiff tongue, dizziness and headache were alleviated, felt warm under feet, Qi and blood unobstructed, the limbs were more comfortable than before, function of the right side hemiparetic limbs resumed to some extent. By Zhang Hua, Innermongolia Sino-mongolian hospital.

Clinical effectiveness statistics:
Sequela of ischemic cerebral disease: 30 cases in the group of pharmacotherapeutic shoes.
Significantly effective: 2 cases, accounted for 6.7% of the total.
Effective: 23 cases, accounted for 76.7% of the total.
Ineffective: 5 cases, accounted for 16.7% of the total.
Total effectiveness 83.3%.
Sequela of ischemic cerebral disease: 30 cases in the control group. Significantly effective: 1 cases, accounted for 3.3% of the total.
Effective: 21 cases, accounted for 70% of the total
Ineffective: 8 cases, accounted for 26.7% of the total
Total effectiveness 73.3%

Conclusion to the clinical trials:
1, for sequela of ischemic cerebral disease, the clinical significant effectiveness and total effectiveness of treatment by using said shoes were 6.7% and 83.3%, respectively, a little higher (3.3% and 73.3%, respectively) than control, however, notwithstanding the statistically significance (P>0.05) between the two groups, the small differences suggested that said shoes can only facilitate to a certain extent the rectification of the dyskinesia of the limbs and abnormal sensation caused by said sequela.
2, said shoes have relatively evident effects of heat insulation as well as improvement of peripheral circulation, it is possible that they could help to promote running of QI of meridian, modulate viscera, Qi and blood in human body, and adjusting the equilibrium of Yin and Yang, thereby facilitating rehabitation.
3, in the course of clinical trials, evident adverse reactions were not found in all of the patients, this suggested that the said shoes are safe.

### X.Clinical trials in the second subsidiary hospital of the Innermongolian medical college

Diseases: prolapse of lumbar intervertebral disc, cervical spondylosis and periarthritis of shoulder.

Starting and finishing dates: 10. Jan - 10. Jul. 1997.

No. of cases: 30 for each kind of disease, 90 patients in total.
① cases of prolapse of lumbar intervertebral disc: male: 15, female: 15, max. age: 68, min. age: 25, Average: 45.1. ② cases of cervical spondylosis: male: 16, female: 14, max. age: 68, min. age: 34, Average: 46.7. ③ cases of periarthritis of shoulder: male: 15, female: 15, max. age: 77, Min. age: 32, Average: 50.1.

Source of the patients: all the 90 patients suffered from 3 diseases were patients being observed by out-patient department in the hospital.

Case analysis: 90 patients were from various cities and mountainous areas in Innermongolia. After putting on said shoes, they basically did not take other therapies. Observation in 2 courses of treatment showed for most of the patients the symptoms faded away to some extent.

Clinical effectiveness rating standard: (Rated into 4 ranks: healed, significantly effective, effective, ineffective, other execution refers to this)

Clinical effectiveness rating standard: for all 3 kinds of the diseases group, rating was divided into significantly effective, effective and ineffective:
1, prolapse of lumbar intervertebral disc:
   ① Significantly effective: all the symptoms and body signs faded away.
   ② Effective: symptom and body signs alleviated evidently.
   ③ Ineffective: no improvement in the symptom and body signs.
2, cervical spondylosis:
   ① Significantly effective: all the symptoms and body signs evidently improved or faded away.
   ② Effective: symptom and body signs are improved to some extent.
   ③ Ineffective: no evident improvement in the symptom and body signs.
3, periarthritis of shoulder:
   ① Significantly effective: all the symptoms and body signs faded away.
   ② Effective: symptom and body signs improved evidently.
   ③ Ineffective: no change in the symptom and body signs.

Typical case: after applying this therapy, symptoms and body signs of most of the 90 patients suffering from the 3 diseases in this group were changed for the better. For some patients the effects were especially evident. For instance: Sun Chunxian, female, 37, suffered from periarthritis in both shoulders, after putting on said shoes, the two lower limbs felt warm first, after 3-4 days all of the symptoms alleviated evidently, after 10 days all the symptoms in two shoulders faded away. Lü minggui, male, 47, Mi Qingkun, female, 42, all suffered from cervical spondylosis, after putting on said shoes, firstly they felt that the functions of their necks flexible and yielding, after 10-14 days, all the symptoms faded away. Other patients had the same recept. As a whole, some patients were especially sensitive to said shoes, they had significant therapeutic effects.

Clinical effectiveness statistics:
1, prolapse of lumbar intervertebral disc: 30 cases
   Significantly effective: 9 cases, accounted for 30% of the total
   Effective: 15 cases, accounted for 50% of the total.
   Ineffective: 6 cases, accounted for 20% of the total.
   Total effectiveness: 80%.
2, cervical spondylosis: 30 cases.
   Significantly effective: 13 cases, accounted for 43% of the total.
   Effective: 13 cases, accounted for 43% of the total.
   Ineffective: 4 cases, accounted for 14% of the total.
   Total effectiveness: 86%.
3, periarthritis of shoulder: 30 cases.
   Significantly effective: 19 cases, accounted for 63% of the total.
   Effective: 8 cases, accounted for 27% of the total.
   Ineffective: 3 cases, accounted for 10% of the total.
   Total effectiveness: 90%.

### Conclusion to the clinical trials:

From the clinical trial and observation in two courses of treatment in 6 months to 90 patients suffering from 3 diseases, it can be certain that this therapy is simple to apply, especially suitable for applications in rural and pasturing areas, it needs not any medical conditions, serving the function of therapy and healthcare only by putting on a pair of shoes. Total effectiveness for 3 diseases was 86%, and no adverse effect have been found, it can be spread for application.

## Claims

1. A shoe for pharmacotherapy, wherein at least one trough (12) is configured in its insole (11), in which arbitrary numbers of containers (13) of arbitrary geometries are placed, said containers (13) are drilled with holes or are covered with fabric (137) from which medicines are leaked out, wherein said containers (13) are loaded with medicinal compositions having pharmacotherapy effect, which comprises at least one of the following categories: powdered preparations, liquid preparations and essential oil compositions, **characterised in that** the bottoms of said containers (13) are having at least one bottom bracket (15) of container (13) to avoid said containers (13) being displaced or dislocated due to the subsidence by trample, wherein said bottom bracket (15) of container (13) is arranged below a part of insole (11) where the corresponding container (13) is located, wherein the top of the bottom bracket (15) of container (13) reaches to the bottom surface of the insole (11).

2. The shoe for pharmacotherapy of claim 1, **characterised in that** the said containers (13) are comprised of the perforated upper lids (131; 134) with inner buttoning flanges or inner arcform buttoning flanges locked down onto the bottom cases (132; 135) with outer buttoning flanges or outer arcform buttoning flanges.

3. The shoe for pharmacotherapy of claim 1, **characterised in that** the said containers (13) are comprised of the retaining rings (133) with inner buttoning flanges or inner arcform buttoning flanges locked down onto the bottom cases (132; 135) with outer buttoning flanges or outer arcform buttoning flanges, the said retaining rings (133) fasten at least one layer of fabric (137) onto the opening of the bottom cases (132; 135).

4. The shoe for pharmacotherapy of claim 1, **characterised in that**; the thickness of the bottom bracket (15) of container (13) varies depending on the thickness of the sole (14), wherein said container (13) and said bottom bracket (15) of container (13) are of any material made into a round shape, a square shape, an unusual shape or any other shape with any volume and any size.

5. The shoe for pharmacotherapy of claim 1, **characterised in that** the said powdered preparations and liquid medicinal preparations include one prepared with single component of borneol, the weight ratio of borneol is in the range from 1 to 9.

6. The shoe for pharmacotherapy of claim 1, **characterised in that** all of the said powdered preparations and liquid preparations include one component borneol, compounded with medicines including clove, amomum cardamomum, angelica, radices zedoariae, radix angelicae and lignum cinnamomi camphorae, or are prepared with any one of the above medicine compounded solely with borneol; the weight ratios of the said medicinal materials clove, amomum cardamomum, angelica, radices zedoariae, radix angelicae and lignum cinnamomi camphorae are in the range from 1 to 9, respectively.

7. The shoe for pharmacotherapy of claim 5 or 6, **characterised in that** the preparation method of said powdered preparations or liquid preparations includes the following procedures:
①weigh out of the materials: weigh out the respective materials according to the compositional weight ratio;
②carefully sort out the respective said materials, grind into fine powder and screen to obtain the powdered drug material;
③borneol is solely added into the powdered drug materials to obtain said single powdered preparation, mixed powdered preparations are prepared by mixing respective single powdered preparation if needed.
④ the above powdered preparations, apart from borneol, are soaked in glacial acetic acid aqueous solution, infiltrated and extracted to obtain stock extract solution respectively, subsequently borneaol ground into fine powder is added into single stock extract solutions to obtain single liquid preparations, wherein a mixed liquid preparation is prepared by mixing single liquid preparations with each other.

8. The shoe for pharmacotherapy of claim 1, **characterised in that** the components of said essential oil compositions include clove oil, eugenol, eugenol type basil oil, eucalyptus oil and borneol, their weight ratios are in the range from 1 to 9, respectively.

9. The shoe for pharmacotherapy of claim 8, **characterised in that** the preparation method of said essential oil compositions includes the following procedures:
①weigh out of the materials: weigh out the respective said essential oil materials according to the compositional weight ratio;
②each of the said essential oil materials is added with borneol, respectively, to obtain single essential oil composition, mixed essential oil compositions are prepared by mixing with each other if needed.

10. The shoe for pharmacotherapy of claim 1, **characterised in that** after mixed up of the said medicament forms, liquid preparations and essential oil compositions, they are applied by dropping into the said empty containers (13); the mixtures of said medicament forms, liquid preparations and essential oil compositions, are applied by dropping into the containers (13) containing said powdered preparations; each of the two said medicament forms, liquid preparations and essential oil compositions, are applied by dropping, respectively, into the containers (13) containing said powdered preparations.

## Patentansprüche

1. Schuh für die Arzneimitteltherapie, wobei wenigstens eine Mulde (12) in seiner Einlage (11) ausgebildet ist, in welcher eine beliebige Anzahl von Behältern (13) mit beliebiger Geometrie angeordnet sind, wobei die Behälter (13) Löcher aufweisen oder mit einem Gewebe (137) bedeckt sind, aus denen Medikamenten werden ausgetreten, wobei die Behälter (13) mit medizinischen Zusammensetzungen mit arzneimitteltherapeutischer Wirkung gefüllt sind und welche wenigstens eine der folgenden Kategorien umfassen: Pulverpräparate, Flüssigpräparate und Zusammensetzungen mit ätherischen Ölen, **dadurch gekennzeichnet, dass** die Unterseiten der Behälter (13) wenigstens eine Bodenstütze (15) des Behälters (13) aufweisen, um zu vermeiden, dass die Behälter (13) aufgrund des Absenkens beim Auftreten verschoben oder verdreht werden, wobei die Bodenstütze (15) des Behälters (13) unter einem Teil der Einlage (11), wo sich der dazugehörige Behälter (13) befindet, angeordnet ist, , wobei sich die Oberseite der Bodenstütze (15) des Behältes (13) bis zur Unterseite der Einlage (11) erstreckt.

2. Schuh für die Arzneimitteltherapie nach Anspruch 1, **dadurch gekennzeichnet, dass** die Behälter (13) perforierte obere Deckel (131, 134) mit innenliegenden Randabschnitten oder bogenförmigen innenliegenden Randabschnitten umfassen, die mit den außenliegenden Randabschnitten oder bogenförmigen außenliegenden Randabschnitten der Bodengehäuse (132, 135) verklemmen.

3. Schuh für die Arzneimitteltherapie nach Anspruch 1, **dadurch gekennzeichnet, dass** die Behälter (13) Halteringe (133) mit innenliegenden Randabschnitten oder innenliegenden bogenförmigen Randabschnitten umfassen, die mit den außenliegenden Randabschnitten oder bogenförmigen Randabschnitten der Bodengehäuse (132, 135) verklemmen, wobei die Halteringe (133) wenigstens eine Gewebelage (137) auf der Öffnung der Bodengehäuse (132, 135) befestigen.

4. Schuh für die Arzneimitteltherapienach Anspruch 1, **dadurch gekennzeichnet, dass** die Dicke der Bodenstütze (15) des Behälters (13) in Abhängigkeit von der Dicke der Sohle (14) variiert, wobei der Behälter (13) und die Bodenstütze (15) des Behältes (13) aus einem beliebigen Material in einer runden Form, einer viereckigen Form, einer unregelmäßigen Form oder jeder anderen Form mit jedem Volumen und jeder Größe gefertigt sind.

5. Schuh für die Arzneimitteltherapienach Anspruch 1, **dadurch gekennzeichnet, dass** die Pulverpräparate und flüssigen medizinischen Präparate ein Präparat umfassen, das mit dem Einzelbestandteil Borneol hergestellt wurde, wobei das Massenverhältnis von Borneol im Bereich von 1 bis 9 liegt.

6. Schuh für die Arzneimitteltherapienach Anspruch 1, **dadurch gekennzeichnet, dass** alle Pulverpräparate und Flüssigpräparate einen Bestandteil Borneol enthalten, gemischt mit Arzneien, die Nelke, Kardamom, Engelwurz, Zittwerwurzel, Angelikawurzel und Kampferbaumholz enthalten, oder mit einer der oben genannten Arzneien hergestellt sind, die ausschließlich mit Borneol gemischt sind, wobei die Massenverhältnisse der medizinischen Substanzen Nelke, Kardamom, Engelwurz, Zittwerwurzel, Angelikawurzel und Kampferbaumholz jeweils im Bereich von 1 bis 9 liegen.

7. Schuh für die Arzneimitteltherapie nach Anspruch 5 oder 6, **dadurch gekennzeichnet, dass** das Herstellungsverfahren der Pulverpräparate und Flüssigpräparate die folgenden Schritte umfasst:
a) Abwiegen der Substanzen: die jeweiligen Substanzen werden entsprechend dem Massenverhältnisses der Zusammensetzung abgewogen,
b) die jeweiligen Substanzen werden vorsichtig sortiert, in feines Pulver gemahlen und ausgesiebt, um das pulverisierte Arzneimittel zu erhalten,
c) Borneol wird einzig zu den pulverisierten Arzneimitteln hinzugefügt, um Einfach-Pulverpräparate zu erhalten, und gemischte Pulverpräparate werden wahlweise hergestellt, indem die jeweiligen Einfach-Pulverpräparate gemischt werden,
d) die oben genannten Pulverpräparate, mit Ausnahme von Borneol, werden in wässriger Eisessiglösung eingeweicht, infiltriert und extrahiert, um die jeweiligen Standardauszugslösungen zu erhalten, anschließend wird in feines Pulver gemahlenes Borneol zu den Einzel-Standardauszugslösungen hinzugefügt, um Einfach-Flüssigpräparate zu erhalten, wobei ein gemischtes Flüssigpräparat durch Vermischen der Einfach-Flüssigpräparate miteinander hergestellt wird.

8. Schuh für die Arzneimitteltherapie nach Anspruch 1, **dadurch gekennzeichnet, dass** die Bestandteile der Zusammensetzungen mit ätherischen Ölen Nelkenöl, Eugenol, Eugenol-haltiges Basilikumöl, Eukalyptusöl und Borneol umfassen, wobei die Massenverhältnisse jeweils im Bereich von 1 bis 9 liegen.

9. Schuh für die Arzneimitteltherapie nach Anspruch 8, **dadurch gekennzeichnet, dass** das Herstellungsverfahren der Zusammensetzungen mit ätherischen Ölen die folgenden Schritte umfasst:
a) Abwiegen der Substanzen: die jeweiligen Substanzen mit ätherischen Ölen werden entsprechend dem Massenverhältnisses der Zusammensetzung abgewogen,
b) Borneol wird zu jeder der ätherischen Ölsubstanzen hinzugefügt, um Einfach-Zusammensetzungen mit ätherischen Ölen zu erhalten, wobei gemischte Zusammensetzungen mit ätherischen Ölen wahlweise durch Vermischen der Einfach-Zusammensetzungen hergestellt werden.

10. Schuh für die Arzneimitteltherapie nach Anspruch 1, **dadurch gekennzeichnet, dass** die Arzneimittelformen, nämlich Flüssigpräparate und Zusammensetzungen mit ätherischen Ölen, nach dem Vermischen verwendet werden, indem sie in die leeren Behälter (13) getropft werden; Gemische dieser Arzneimittelformen, nämlich Flüssigpräparate und Zusammensetzungen mit ätherischen Ölen, verwendet werden, indem sie in die Behälter (13) getropft werden, welche Pulverpräparate enthalten; jede der beiden Arzneimittelformen, nämlich Flüssigpräparate und Zusammensetzungen mit ätherischen Ölen, verwendet werden, indem sie jeweils in die Behälter (13) getropft werden, welche Pulverpräparate enthalten.

## Revendications

1. Chaussure pour pharmacothérapie, dans laquelle au moins un creux (12) est configuré dans la semelle intérieure (11) de celle-ci, dans lequel un nombre arbitraire de récipients (13) à géométries arbitraires sont placés, lesdits récipients (13) étant percés de trous ou étant recouverts de tissus (137) d'où s'échappent des médicaments,
dans laquelle lesdits récipients (13) sont remplis de compositions médicinales ayant un effet de pharmacothérapie, lesquelles comprennent au moins l'une des catégories suivantes : des préparations pulvérulentes, des préparations liquides et des compositions d'huiles essentielles,
**caractérisée en ce que** les fonds desdits récipients (13) possèdent au moins un support de fixation de fond (15) de récipient (13) afin d'éviter que lesdits récipients (13) se déplacent ou se disloquent du fait de l'affaissement par piétinement, dans laquelle ledit support de fixation de fond (15) de récipient (13) est disposé en dessous d'une partie de la semelle intérieure (11) où le récipient (13) correspondant se trouve, dans laquelle le haut du support de fixation de fond (15) de récipient (13) atteint la surface inférieure de la semelle intérieure (11).

2. Chaussure pour pharmacothérapie selon la revendication 1, **caractérisée en ce que** lesdits récipients (13) sont constitués par les couvercles supérieurs perforés (131 ; 134) avec des rebords de boutonnage intérieurs ou des rebords de boutonnage intérieurs en forme d'arc verrouillés sur les réceptacles inférieurs (132 ; 135) avec des rebords de boutonnage extérieurs ou rebords de boutonnage extérieurs en forme d'arc.

3. Chaussure pour pharmacothérapie selon la revendication 1, **caractérisée en ce que** lesdits récipients (13) sont constitués par les bagues de retenue (133) avec les rebords de boutonnage intérieurs ou rebords de boutonnage intérieurs en forme d'arc verrouillés sur les réceptacles inférieurs (132 ; 135) avec des rebords de boutonnage extérieurs ou rebords de boutonnage extérieurs en forme d'arc, lesdites bagues de retenue (133) fixant au moins une couche de tissu (137) sur l'ouverture des réceptacles inférieurs (132 ; 135).

4. Chaussure pour pharmacothérapie selon la revendication 1, **caractérisée en ce que** l'épaisseur du support de fixation de fond (15) de récipient (13) varie en fonction de l'épaisseur de la semelle (14), dans laquelle ledit récipient (13) et ledit support de fixation de fond (15) de récipient (13) sont en un matériau quel qu'il soit auquel on a conféré une forme ronde, une forme carrée, une forme inhabituelle ou toute autre forme avec un volume et une taille quels qu'ils soient.

5. Chaussure pour pharmacothérapie selon la revendication 1, **caractérisée en ce que** lesdites préparations pulvérulentes et préparations médicinales liquides comprennent une préparation préparée avec un composant simple de bornéol, le rapport pondéral du bornéol se situant dans la plage de 1 à 9.

6. Chaussure pour pharmacothérapie selon la revendication 1, **caractérisée en ce que** l'ensemble desdites préparations pulvérulentes et préparations liquides comprennent un composant de bornéol mélangé avec des remèdes comprenant du clou de girofle, de l'amomum cardamomum, de l'angélique, du radices zedoaria, du radix angelicae et du lignum cinnamomi camphorae, ou sont préparées avec l'un quelconque des remèdes ci-dessus mélangé seulement avec du bornéol ; les rapports pondéraux desdites substances médicinales que sont le clou de girofle, l'amomum cardamomum, l'angélique, le radices zedoaria, le radix angelicae et le lignum cinnamomi camphorae, se situant respectivement dans la plage de 1 à 9.

7. Chaussure pour pharmacothérapie selon la revendication 5 ou 6, **caractérisée en ce que** le procédé de préparation desdites préparations pulvérulentes ou préparations liquides comprend les procédures suivantes ;
1) peser les substances : peser les substances respectives en fonction du rapport pondéral de composition ;
2) trier avec précaution lesdites substances respectives, les broyer en une poudre fine et les tamiser pour obtenir la substance médicamenteuse pulvérulente ;
3) le bornéol est seulement ajouté aux substances médicamenteuses pulvérulentes pour obtenir ladite préparation pulvérulente simple, les préparations pulvérulentes mélangées sont préparées en mélangeant respectivement des préparations pulvérulentes simples si nécessaire,
4) les préparations pulvérulentes ci-dessus, mis à part le bornéol, sont respectivement trempées dans une solution aqueuse glacée d'acide acétique, soumises à infiltration et extraites pour obtenir une solution mère d'extrait, ensuite, du bornéol broyé en poudre fine est ajouté dans des solutions mères d'extrait simples pour obtenir des préparations liquides simples, dans laquelle une préparation liquide mélangée est préparée par mélange les des préparations liquides simples les unes avec les autres.

8. Chaussure pour pharmacothérapie selon la revendication 1, **caractérisée en ce que** les composants desdites compositions d'huiles essentielles comprennent de l'huile de clou de girofle, de l'eugénol, de l'huile de basilic de type eugénol, de l'huile d'eucalyptus et du bornéol, leurs rapports pondéraux se situant respectivement dans la plage de 1 à 9.

9. Chaussure pour pharmacothérapie selon la revendication 8, **caractérisée en ce que** le procédé de préparation desdites compositions d'huiles essentielles comprend les procédures suivantes :
1) peser les substances : peser lesdites substances d'huiles essentielles respectives en fonction du rapport pondéral de composition ;
2) ajouter du bornéol respectivement dans chacune desdites substances d'huiles essentielles pour obtenir une composition d'huile essentielle simple, les compositions d'huiles essentielles mélangées étant préparées par mélange les unes avec les autres si nécessaire.

10. Chaussure pour pharmacothérapie selon la revendication 1, **caractérisée en ce que**, après avoir mélangé lesdites formes médicamenteuses, préparations liquides et compositions d'huiles essentielles, elles sont appliquées en les faisant tomber goutte à goutte à l'intérieur desdits récipients (13) ; les mélanges desdites formes médicamenteuses, préparations liquides et compositions d'huiles essentielles étant appliqués en les faisant tomber goutte à goutte à l'intérieur des récipients (13) contenant lesdites préparations pulvérulentes ; chacune desdites deux formes médicamenteuses, préparations liquides et compositions d'huiles essentielles étant appliquées en les faisant respectivement tomber goutte à goutte à l'intérieur des récipients (13) contenant lesdites préparations pulvérulentes.
